# EUROPÄISCHE PATENTANMELDUNG

(11) **EP 0 882 440 A2**
(43) Veröffentlichungstag der Anmeldung: **09.12.1998**
(21) Anmeldenummer: 98109305.7
(22) Anmeldetag: 22.05.1998
(51) Int. Cl.: A61J 1/00, B65D 47/26, A61L 11/00, A61M 1/00

(54) **Absaugvorrichtung für Körperflüssigkeiten**

(30) Priorität: 03.06.1997 DE 19723197
(71) Anmelder: B. BRAUN MELSUNGEN AG, 34212 Melsungen (DE)
(72) Erfinder: Koetke, Claus-Dieter, 29394 Reinstorf/Lüder (DE); Sippel, Martin, Dr., 34212 Melsungen (DE); Klute, Volker, 34212 Melsungen (DE)
(74) Vertreter: Selting, Günther, Dipl.-Ing.

(57) **Zusammenfassung**

Eine Absaugvorrichtung für Körperflüssigkeiten weist einen Behälter (10) auf, der mit einem Deckel (11) luftdicht verschließbar ist. An einen zylindrischen Ansatz (12) des Deckels (11) ist mittels eines Halterings (13) ein Beutel (14) befestigt. In dem Deckel (11) ist eine Saugöffnung (15) vorgesehen, um in dem Beutel (14) Unterdruck zu erzeugen. Ferner weist die Saugöffnung (15) eine Abzweigung (31) auf, um gleichzeitig in dem Behälter (10) Unterdruck zu erzeugen. Zusätzlich weist der Deckel (11) eine Auslaßöffnung (17) auf, die von einem Hohlkörper (37) verschlossen ist. Der Hohlkörper (37) ist zum Entleeren des Beutels (14) in den Behälter (10) eindrückbar, so daß die in dem Beutel (14) enthaltene Flüssigkeit über Öffnungen (40,41) des Hohlzylinders (37) ausgießbar ist.

## Beschreibung

Die Erfindung betrifft eine Absaugvorrichtung für Körperflüssigkeiten, insbesondere zum Einsatz in Krankenhäusern.

Absaugvorrichtungen, wie beispielsweise in EP 0 390 094 A1 beschrieben, weisen einen festen Behälter auf, in den ein flexibler Beutel eingesetzt ist. Der Beutel ist an einem Deckel gehalten, mit dem der Behälter luftdicht verschließbar ist. Ferner sind an dem Deckel zwei Anschlüsse vorgesehen, von denen einer an den Patienten und der andere an eine Saugquelle anschließbar ist. Beide Anschlüsse weisen in das Beutelinnere. Um zu verhindern, daß der Beutel von der Saugquelle vollständig zusammengesaugt wird, so daß er keine Flüssigkeit mehr aufnehmen kann, muß der Behälter ebenfalls an eine Saugquelle angeschlossen sein. Somit herrscht sowohl in dem Beutel als auch in dem Behälter Unterdruck, so daß die Form des Beutels erhalten bleibt und diese Körperflüssigkeiten aufnehmen kann.

Sobald der Beutel mit Körperflüssigkeit gefüllt ist, muß diese beispielsweise durch Ausgießen in die Kanalisation entsorgt werden. Dabei muß darauf geachtet werden, daß die Körperflüssigkeit häufig kontaminiert ist und daher vermieden werden muß, daß das Personal mit der Flüssigkeit in Kontakt kommt. Um dies zu vermeiden, werden häufig die geschlossenen Beutel durch Naßverbrennung entsorgt. Dieses Vorgehen ist äußerst kostenintensiv und aus hygienischen Gründen für Absauggut aus meldepflichten Krankheiten vorgeschrieben.

Ferner besteht die Möglichkeit, der in dem Beutel vorhandenen Körperflüssigkeit ein Eindickungsmittel zuzuführen, um die Flüssigkeit einzudicken. Die eingedickte Flüssigkeit kann anschließend deponiert oder ebenfalls verbrannt werden. Durch das Eindicken der Flüssigkeit ist das Kontaminierungsrisiko des Personals verringert, weil die Gefahr durch unkontrolliert austretende Flüssigkeit, die beim Ausgießen nicht vermieden werden kann, entfällt. Allerdings besteht beim Zuführen des Eindickungsmittels sowie beim Entnehmen des Beutels aus dem Behälter weiterhin eine Kontaminierungsgefahr, wenn die Flüssigkeit beispielsweise nicht vollständig eingedickt wird. Außerdem ist das Eindicken von Körperflüssigkeiten zeit- und kostenintensiv.

Als dritte, äußerst kostenintensive Entsorgungsmöglichkeit kann der komplette Behälter im Wege der Naßverbrennung entsorgt werden.

Aufgabe der Erfindung ist es, eine Absaugvorrichtung für Körperflüssigkeiten zu schaffen, bei der die Kontaminierungsgefahr auch beim Ausgießen verhindert ist.

Die Lösung der Aufgabe erfolgt erfindungsgemäß durch die Merkmale des Patentanspruchs 1.

Erfindungsgemäß ist in einer Auslaßöffnung der Absaugvorrichtung ein Schieber angeordnet, der zum Öffnen drehbar oder axial bewegbar ist. Um den drehbaren Schieber zum Einstreuen von Eindickungsmittel zu öffnen, wird der Behälter so gehalten, daß die Auslaßöffnung nach oben weist und der Schieber um einen bestimmten Winkel verdreht wird. Durch das Verdrehen des Schiebers gelangen in dem Schieber vorgesehene Öffnungen mit in dem Behälter vorgesehenen Öffnungen zur Deckung, so daß die in dem Behälter befindliche Körperflüssigkeit durch Einstreuen von Eindickungsmitteln eingedickt werden kann. Bei einer zweiten Öffnungsmöglichkeit des verdrehbaren Schiebers zum Ausgießen der Flüssigkeit wird der Behälter bei geschlossenem Schieber auf dem Kopf stehend bzw. mit nach untem gerichteter Auslaßöffnung auf eine am Beckenboden eines Ausgußbeckens befestigte Öffnungshilfe aufgesetzt. Die Öffnungshilfe greift in den Schieber ein, so daß die Auslaßöffnung durch Drehen des Behälters geöffnet werden kann. Somit muß der drehbare Schieber zum Öffnen vom Personal nicht berührt werden und es besteht keinerlei Kontaminierungsgefahr.

Der axial bewegbare Schieber ragt in der Verschlußstellung aus dem Behälter hervor und wird zum Öffnen in den Behälter in axiale Richtung eingedrückt. Zum Eindrücken des Schiebers bestehen ebenfalls zwei Möglichkeiten.

Bei der ersten Möglichkeit wird der Behälter so gehalten, daß die Auslaßöffnung nach oben weist und somit beim Eindrücken des Schiebers keine Körperflüssigkeit aus dem Behälter austreten kann. Anschließend kann das Eindickungsmittel in den Behälter eingestreut werden. Es ist nicht erforderlich den Schieber vor dem Eindrücken zu drehen oder anderweitig zu entriegeln. Ferner kann der Schieber mit Hilfe eines Gegenstands eingedrückt werden, so daß ein großer Abstand zur Körperflüssigkeit besteht.

Da der Schieber in der Verschlußstellung den Behälter abdichtet, kann der Behälter zum Öffnen als zweite Möglichkeit mit nach unten weisender Auslaßöffnung gehalten werden. In dieser Lage wird der Schieber beispielsweise gegen den Boden eines Ausgußbeckens o.dgl. gedrückt, so daß der Schieber in den Behälter geschoben wird. Auf dem Kopf stehend bzw. mit nach unten gerichteter Auslaßöffnung kann die Flüssigkeit aus dem Beutel auslaufen. Hierbei ist es zu keinem Zeitpunkt erforderlich, daß das Personal den Schieber berührt.

Vorzugsweise sind der drehbare und der axial bewegbare Schieber als behälterseitig geschlossener Hohlkörper ausgebildet. Der Hohlkörper weist seitlich mindestens eine Öffnung auf. In der Verschlußstellung des drehbaren Schiebers ist diese Öffnung von einem Ansatz des Behälters bedeckt und somit verschlossen. Bei dem axial bewegbaren Schieber ist diese Öffnung außerhalb des Behälters bzw. außerhalb der durch die behälterseitige Fläche des Hohlkörpers gebildeten Dichtfläche angeordnet. Ferner weist der Hohlkörper an einer dem Behälter abgewandten Seite eine Ausgußöffnung auf, so daß bei geöffnetem Schieber die Körperflüssigkeit durch die seitliche Öffnung in den Hohlkörper fließt und aus diesem durch die Ausgußöffnung ausgießbar ist. Die behälterseitige Fläche des Hohlkörpers kann als Führungskörper zum Leiten der Flüssigkeit und als zusätzlicher Spritzschutz ausgebildet sein. Aufgrund der Ausgestaltung des Schiebers dient er sowohl zum Öffnen der Auslaßöffnung ohne Kontaminierungsgefahr als auch zum Verbessern der Ausgießeigenschaften.

Vorzugsweise endet der axial bewegbare Schieber in eingedrücktem Zustand in gleicher Höhe wie die beiden Anschlußstutzen von Saug- und Einlaßöffnung, so daß der Behälter auf diesen drei Stützen auf einer ebenen Fläche stabil stehen kann. Somit kann der Behälter zum Entleeren einfach in eine entsprechende Auffangwanne gestellt werden und muß nicht vom Personal gehalten werden. Bei einem Behälter mit drehbarem Schieber ist die Höhe der auf dem Beckenboden befestigten Öffnungshilfe zum Öffnen des drehbaren Schiebers so mit dem drehbaren Schieber abgestimmt, daß auch der Behälter mit drehbarem Schieber auf den beiden Anschlußstutzen und dem Schieber sicher steht und nicht vom Personal gehalten werden muß.

Da in dem Behälter und in dem Beutel während des Betriebs der Absaugvorrichtung Unterdruck herrscht, muß bei dem axial bewegbaren Schieber sichergestellt sein, daß der Schieber aufgrund des Unterdrucks nicht aus seiner Verschlußstellung in den Behälter gezogen werden kann. Hierbei würde die Auslaßöffnung des Behälters geöffnet, so daß in dem Beutel kein Unterdruck mehr erzeugt werden kann und demzufolge von der Absaugvorrichtung keine Körperflüssigkeit mehr abgesaugt wird. Um dies zu vermeiden, kann der Schieber beispielsweise in der Verschlußstellung leicht verdreht werden, so daß ein Öffnen des Schiebers aufgrund von Unterdruck nicht möglich ist. Allerdings müßte der Schieber dann vor dem Eindrücken wieder zurückgedreht werden.

Vorzugsweise ist daher eine Kappe vorgesehen, die den Schieber umschließt und mit dem Behälter luftdicht abschließt. Würde der Schieber nun aufgrund des Unterdrucks geringfügig in den Behälter gezogen, so entsteht ein Spalt zwischen dem Schieber und einer entsprechenden Dichtfläche an der Auslaßöffnung, so daß in der den Schieber umschließenden Kappe ebenfalls Unterdruck erzeugt wird. Da die Kappe den Behälter luftdicht verschließt, herrscht auf beiden Seiten des Schiebers der gleiche Druck, so daß der Schieber nicht weiter in den Behälter gezogen wird. Somit ist auf einfache Weise sichergestellt, daß der Schieber nicht durch den in dem Beutel herrschenden Unterdruck in den Behälter gezogen wird.

Gemäß einer Weiterentwicklung, die selbständige Bedeutung hat, ist der Behälter mit einem Deckel luftdicht verschließbar, an dem der Beutel befestigt ist. In dem Deckel ist die Saug-, die Einlaß- und die Auslaßöffnung vorgesehen. Erfindungsgemäß weist die mit dem Beutel verbundene Saugöffnung eine in dem Deckel vorgesehene Abzweigung in den Behälter auf. Somit besteht von der Saugöffnung einerseits eine Verbindung in den Beutel und andererseits über die Abzweigung eine Verbindung in den Behälter. Somit wird mittels der an die Saugöffnung angeschlossenen Saugquelle sowohl in dem Beutel als auch in dem Behälter Unterdruck erzeugt. Es ist daher nicht erforderlich, zwei Schläuche anzuschließen, von denen einer mit dem Beutel und der andere mit dem Behälter verbunden ist. Vielmehr muß zum Erzeugen des Unterdrucks in dem Beutel und dem Behälter lediglich ein Schlauch angeschlossen werden.

Auch bei einer Anordnung von mehreren Absaugvorrichtungen in einer Reihe muß lediglich die Einlaßöffnung eines nachfolgenden Behälters mit der Saugöffnung eines vorhergehenden Behälters verbunden werden. Ein zusätzliches Anschließen jedes Behälters mittels eines weiteren Schlauchs an die Saugquelle ist nicht erforderlich.

Bei in Reihe geschalteten Behältern ist ferner die Einlaßöffnung des patientenseitigen ersten Behälters mit dem Patienten und die Saugöffnung des letzten Behälters mit der Saugquelle verbunden. Hierbei ist in der Saugöffnung des letzten Behälters ein hydrophober Filter angeordnet, der die Saugöffnung verschließt sobald er mit Feuchtigkeit in Berührung kommt. Somit ist sichergestellt, daß keine Flüssigkeit in die Saugvorrichtung gelangt. Die Saugöffnungen der übrigen Behälter sind jeweils mit einem Rückschlagventil versehen, um zu vermeiden, das Körperflüssigkeit in einen vorhergehenden Behälter zurückfließt.

Nachfolgend wird die Erfindung anhand einer bevorzugten Ausführungsform unter Bezugnahme auf die anliegenden Zeichnungen näher erläutert.

Es zeigen:
- Fig. 1: eine schematische Seitenansicht einer bevorzugten Ausführungsform einer Absaugvorrichtung;
- Fig. 2: eine Draufsicht auf die Absaugvorrichtung;
- Fig. 3: einen Längsschnitt der Absaugvorrichtung mit axial bewegbarem Schieber bei geschlossener Auslaßöffnung;
- Fig. 4: einen Längsschnitt der Absaugvorrichtung mit axial bewegbarem Schieber bei geöffneter Auslaßöffnung;
- Fig. 5: ein Längsschnitt der Absaugvorrichtung mit drehbarem Schieber bei geschlossener Auslaßöffnung;
- Fig. 6: ein Längsschnitt der Absaugvorrichtung mit drehbarem Schieber bei geöffneter Auslaßöffnung, und
- Fig. 7: eine Anordnung zweier Absaugvorrichtungen in Reihe.

Die Absaugvorrichtung für Körperflüssigkeiten weist einen starren Behälter 10 auf, der mittels eines Deckels 11 luftdicht verschließbar ist. Der Deckel 11 weist einen zylindrischen Ansatz 12 auf, der in den Behälter 10 vorsteht. Am äußeren Umfang des zylindrischen Ansatzes 12 ist mittels eines Halterings 13 ein flexibler Beutel 14 befestigt. Der Beutel 14 ist somit fest und abdichtend an dem Deckel 11 gehalten und erstreckt sich von dem zylindrischen Ansatz 12 des Deckels in den Behälter 10.

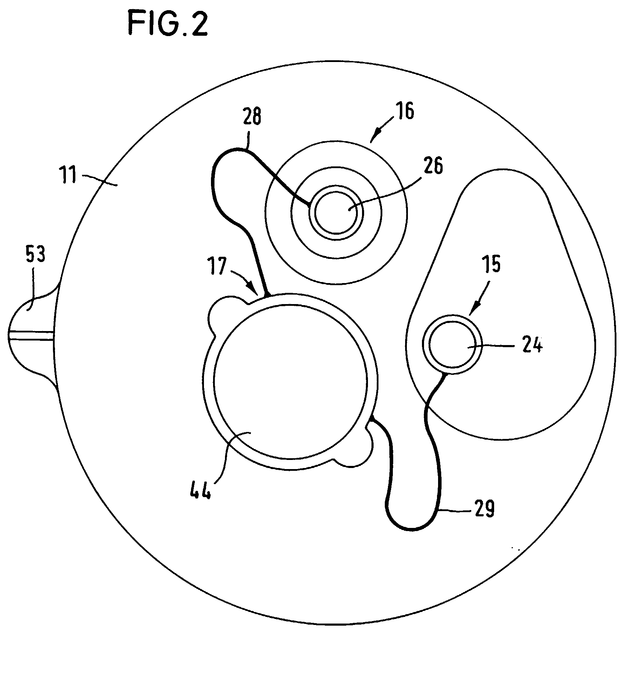

In dem Deckel 11 ist eine Saugöffnung 15 vorgesehen, die an eine nicht dargestellte externe Saugquelle anschließbar ist. Ferner weist der Deckel 11 eine Einlaßöffnung 16 (Fig. 2) auf, an die ein von einem Patienten kommender Schlauch anschließbar ist. Die Auslaßöffnung 15 und die Einlaßöffnung 16 führen in den zylindrischen Ansatz 12 des Deckels 11 und somit in den Beutel 14, in dem die von dem Patienten abgesaugte Körperflüssigkeit gesammelt wird. Des weiteren ist in dem Deckel 11 eine Auslaßöffnung 17 derart angeordnet, daß eine Verbindung mit dem zylindrischen Ansatz 12 und somit mit dem Beutel 14 besteht. Die Auslaßöffnung 17 dient zum Entleeren des Beutels 14.

Im Bereich der Saugöffnung 15 ist mit dem Deckel 11 ein Filterdeckel 21 verbunden, der eine mit der in dem Deckel 11 vorgesehenen Saugöffnung 15 fluchtende Öffnung 22 aufweist. Koaxial zu der Öffnung 22 ist an dem Deckel 21 ein Anschlußstutzen 23 angeordnet, auf den beispielsweise zum Transport eine Verschlußkappe 24 aufsteckbar ist. Während des Betriebs der Absaugvorrichtung ist die Verschlußkappe 24 von dem Ansatzstutzen 23 abgezogen und statt dessen ein Schlauch auf den Ansatzstutzen 23 aufgesteckt, der zu einer Saugquelle führt.

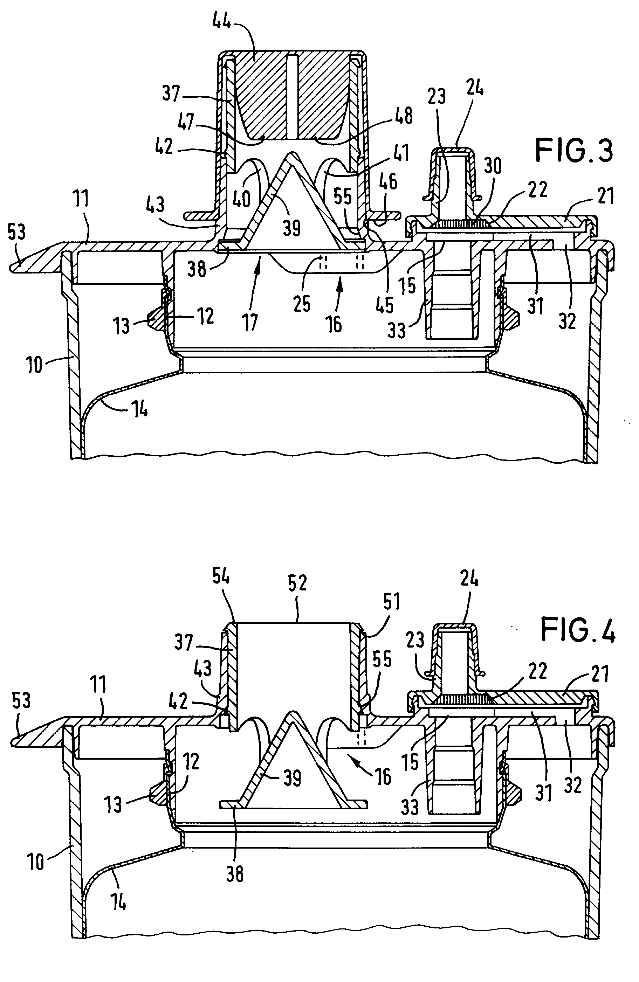

Entsprechend weist der Deckel 11 im Bereich der Einlaßöffnung einen Anschlußstutzen 25 (Fig. 3) auf, auf den entweder eine Verschlußkappe 26 (Fig. 2) oder während des Betriebs der Absaugvorrichtung ein an einen Patienten anschließbarer Schlauch aufgesteckt ist. Die Verschlußkappen 24,26 sind mittels Bändern 28,29 an dem Deckel 11 befestigt.

Gemäß Fig. 3 ist in der Öffnung 22 des Filterdeckels 21 ein hydrophober Filter 30 vorgesehen. Dieser ist luftdurchlässig, wird aber sobald er mit Feuchtigkeit in Berührung kommt luftundurchlässig. In dem Bereich des Deckels 11 unterhalb des Filterdeckels 21 ist in dem Deckel 11 eine Nut vorgesehen, die bei geschlossenem Filterdeckel 21 eine Abzweigung 31 von der Saugöffnung 15 bildet. Am in Fig. 3 rechten Ende der Abzweigung 31 ist in dem Deckel 11 eine Öffnung 32 vorgesehen, die die Abzweigung 31 mit dem Inneren des Behälters 10 verbindet. Ist an den Anschlußstutzen 23 mittels eines Schlauchs eine Saugquelle angeschlossen, so wird über die Saugöffnung 15 in dem Beutel 14 Unterdruck erzeugt, indem über einen Ansatz 33, der zu der Saugöffnung 15 koaxial an dem Deckel 11 vorgesehen ist und in den zylindrischen Ansatz 12 des Deckels 11 ragt, aus dem Beutel Luft gesaugt wird. Gleichzeitig wird über die Abzweigung 31 und die Öffnung 32 aus dem den Beutel 14 umgebenden Behälter 10 Luft gesaugt, so daß sowohl in dem Beutel 14 als auch in dem Behälter 10 annähernd gleicher Unterdruck herrscht.

In der in Fign. 3 und 4 dargestellten Ausführungsform ist in der Auslaßöffnung 17 als axial bewegbarer Schieber 37 ein zylindrischer Hohlkörper angeordnet. Der zylindrische Hohlkörper 37 ist behälterseitig von einer Stirnwand 38 verschlossen, die in der Verschlußstellung die Auslaßöffnung 17 abdichtet. Die Stirnwand 38 weist eine in das Innere des Hohlzylinders 37 aufragende Einbuchtung auf, die einen kegelförmigen Führungskörper 39 bildet. Im Höhenbereich des kegelförmigen Führungskörpers sind in der Seitenwand des Hohlzylinders 37 zwei Öffnungen 40,41 vorgesehen.

Ferner weist der Hohlzylinder 37 eine am Umfang angeordnete umlaufende Rastnase 42 auf. In der in Fig. 3 dargestellten Verschlußstellung des Hohlzylinders 37 liegt die Rastnase 42 an der nach außen weisenden Fläche eines Ansatzstutzens 43 an. Der Ansatzstutzen 43 ist an dem Deckel 11 koaxial zu der Auslaßöffnung 17 angeordnet. Er dient zur Führung des Hohlzylinders 37 und verdeckt in der Verschlußstellung die Öffnungen 40,41.

Über den Ansatzstutzen 43 ist eine Kappe 44 gesteckt, die den Hohlzylinder 37 umschließt, wobei die Innenseite der Kappe 44 an der Außenseite des Stutzens 43 anliegt. Damit die Lage der Kappe 44 genau festgelegt ist und die Kappe 44 nicht weiter in Richtung des Deckels 11 bewegt werden kann, weist der Ansatzstutzen 43 einen umlaufenden Absatz 45 auf, an dem die Unterkante 46 der Kappe 44 anliegt. Ferner weist die Kappe 44 Rippen 47,48 auf, die in den Hohlzylinder hineinragen. Die Rippen 47,48, die durch weitere sternförmig angeordnete Rippen ergänzt sein können, dienen zum genauen Aufsetzen der Kappe 44 auf den Hohlzylinder 37, damit der Hohlzylinder 37 beim Aufsetzen der Kappe 44 nicht in den Behälter 10 gedrückt wird. Somit umschließt die Kappe 44 den Hohlzylinder 37 und dichtet gegenüber dem Ansatzstutzen 33 des Deckels 11 ab.

Wird zum Betreiben der Absaugvorrichtung auf den Anschlußstutzen 23 der Saugöffnung 15 ein an eine Saugquelle angeschlossener Schlauch aufgesteckt, so wird von der Saugquelle im Behälter 10 und in dem Beutel 14 Unterdruck erzeugt. Aufgrund des Unterdrucks wirkt auf den Hohlzylinder 37 eine in Fig. 3 nach unten gerichtete Kraft. Hierdurch kann der Hohlzylinder 37 in Fig. 3 geringfügig nach unten verschoben werden, bis zwischen der Stirnfläche 38 und der Dichtfläche des Ansatzstutzens 43 ein geringer Spalt entsteht. Aufgrund dieses Spalts wird in dem Hohlzylinder 37 ebenfalls Unterdruck erzeugt, so daß in dem Hohlzylinder 37 und in dem Beutel 14 der gleiche Druck herrscht. Da die Kappe 44 abdichtend auf den Ansatzstutzen 43 aufgesteckt ist und den Hohlzylinder 37 umschließt, herrscht auch in der Kappe 44 derselbe Unterdruck. Somit ist verhindert, daß der Hohlzylinder 37 aufgrund des in dem Beutel 14 herrschenden Unterdrucks in den Behälter 10 gezogen wird. Da aufgrund der umlaufenden Rastnase 42 des Hohlzylinders 37 dieser nach dem Druckausgleich wieder in die Verschlußstellung zurückgebracht wird, befindet sich der Hohlzylinder 37 während des Betriebs der Absaugvorrichtung stets in Verschlußstellung.

Sobald der Beutel 14 voll ist oder aus einem anderen Grund entleert werden muß, wird die Saugquelle abgestellt und die an die Einlaß- bzw. Saugöffnung angeschlossenen Schläuche werden von den Anschlußstutzen 25 bzw. 23 abgezogen. Um beim Abziehen des Patientenschlauchs ein Berühren mit Körperflüssigkeiten zu vermeiden, kann das freie Ende des Patientenschlauchs auch auf den Anschlußstutzen 23 der Saugöffnung gesteckt werden. Sobald der Unterdruck in dem Beutel 14 nachläßt, wird der Hohlzylinder 37 von der Rastnase 42 wieder in seine Verschlußstellung gebracht und der Behälter 10 kann beispielsweise zum Entleeren der in dem Beutel 14 gesammelten Körperflüssigkeit ohne Risiko zum Fäkalienraum transportiert werden.

Ist der Beutel mit Körperflüssigkeit aus meldepflichtigen Krankheiten gefüllt, werden die Ansaugstutzen 23,25 und der Hohlzylinder 37 mit den Kappen 24,26 und 44 verschlossen, so daß beim Transport keine Flüssigkeit auslaufen kann. Anschließend wird der gesamte Behälter 10 durch Naßverbrennung entsorgt.

Zum Entleeren des Beutels 14 wird die Kappe 44 von dem Ansatzstutzen 43 abgezogen. Der Hohlzylinder befindet sich weiterhin in der Verschlußstellung, so daß der Behälter dicht verschlossen ist. Anschließend wird der Hohlzylinder 37, wie in Fig. 4 dargestellt, zum Öffnen in den Behälter 10 eingedrückt. Dazu wird die Absaugvorrichtung am Behälter 10 oder am Beutel 14, also mit ausreichendem Sicherheitsabstand zur Ausgußöffnung gehalten und mit dem Deckel 11 nach unten weisend auf den Boden des Ausgußbeckens gedrückt. Der Hohlzylinder 37 kann so weit in den Behälter 10 eingedrückt werden, bis ein vom Behälter abgewandten Ende des Hohlzylinders 37 vorgesehener Ansatz 51 an der Oberseite des Ansatzstutzens 43 anliegt. Aufgrund der kegeligen Form des Führungskörpers 39 gelangt selbst dann, wenn der Schieber 37 beim Eindrücken in den Behälter 10 in Flüssigkeit eintaucht, die Flüssigkeit nicht schlagartig in den Hohlzylinder 37, so daß ein Kontakt des Personals mit ggf. kontaminierter Flüssigkeit vermieden ist. Zum Ausgießen der in dem Beutel 14 vorhandenen Flüssigkeit muß der Behälter 10 nun lediglich geneigt werden, so daß die Flüssigkeit durch die Öffnungen 40,41 in dem Hohlzylinder 37 in diesen strömen kann und durch eine Ausgußöffnung 52 des Hohlzylinders 37 ausgießbar ist. Hierbei kann der Behälter an der dem Deckel 11 gegenüberliegenden Seite gehalten werden, so daß der Kontakt mit Körperflüssigkeit wiederum vermieden ist. Da die Anschlußstutzen 23,25 mit dem Hohlzylinder 37 in geöffneter Stellung in gleicher Höhe abschließen, kann die Absaugvorrichtung zum Entleeren auch einfach mit nach unten weisender Auslaßöffnung 52 in eine Auffangwanne gestellt werden. Zum Ablaufen der Flüssigkeit muß der Behälter 10 dann lediglich seitlich verschoben werden, so daß die Auslaßöffnung des Deckels 11 über der Abflußöffnung des Ausgußbeckens liegt.

Zum Entleeren des Beutels 14 kann auch der Deckel 11 mit Hilfe einer Lasche 53 von dem Behälter 10 abgenommen werden. Durch das Anheben des Deckels 11 wird der Beutel 14, der über den Haltering 13 mit dem zylindrischen Ansatz ]2 des Deckels fest verbunden ist, aus dem Behälter 10 gezogen. Zum Transport des Beutels 14 weist dieser an der dem Deckel 11 gegenüberliegenden Unterseite eine oder mehrere nicht dargestellte Tragebügel auf, an denen der Beutel mit nach unten weisendem Deckel gehalten werden kann. Da auch bei nach unten weisendem Deckel 11 die Stirnwand 38 des Hohlzylinders 37 die Auslaßöffnung 17 abdichtet, kann auch in dieser Lage die Kappe 44 von dem Ansatzstutzen 43 abgezogen werden, ohne daß das Personal mit Körperflüssigkeit in Berührung kommt. Der Hohlzylinder 37 wird in den Beutel 14 eingedrückt, indem die vom Deckel 11 abgewandte Stirnseite 54 des Hohlzylinders 37 kurz, beispielsweise auf den Boden der Auffangwanne aufgesetzt wird. Der Ansatzstutzen 43 weist eine Abschrägung 55 auf, an der die Rastnase 42 in geöffneter Stellung einrastet, so daß der Hohlzylinder 37 beim Ausgießen nicht in die Verschlußstellung zurückgedrückt wird.

Findet ein Entleeren des Beutels 14 beispielsweise aus Sicherheitsgründen nicht statt, so kann der Beutel 14 zusammen mit dem Deckel 11 entsorgt werden. Der Behälter 10 hingegen kann wieder verwendet werden.

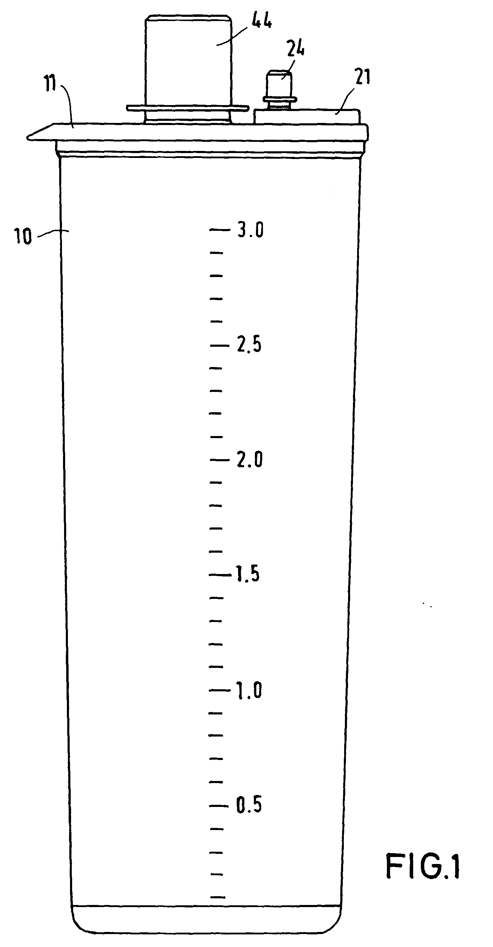

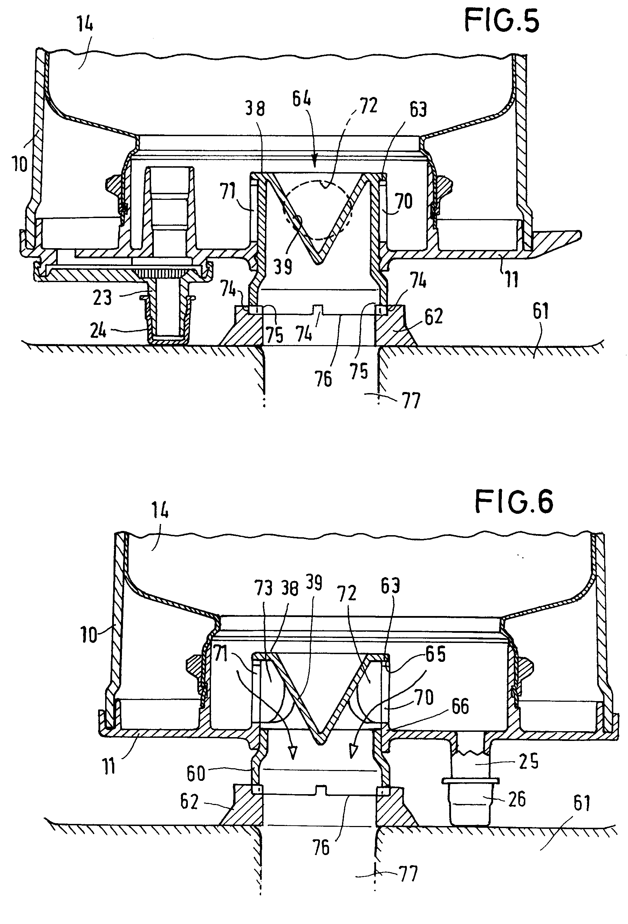

In den Fign. 5 und 6 ist eine Ausführungsform mit drehbarem Schieber 60 dargestellt. Mit Ausnahme der Funktion des Schiebers entsprechen sämtliche Anschlüsse und Funktionen der anhand der Fign. 1 bis 4 beschriebenen Ausführungsform. Der Behälter 10 ist in Fign. 5 und 6 auf dem Kopf stehend bzw. mit nach untem weisenden Deckel 11 dargestellt. Die Absaugvorrichtung steht auf einem Beckenboden 61, in dem die Verschlußkappe 24 des Ansatzstutzens 23 und die Verschlußkappe 26 des Ansatzstutzens 25 (Fig. 6) auf dem Beckenboden 61 aufliegen sowie der Hohlzylinder 60 auf einer Öffnungshilfe 62 aufliegt.

Der Hohlzylinder 60 ist behälterseitig von einer Stirnwand 63 verschlossen, die in der in Fig. 5 dargestellten Verschlußstellung die Auslaßöffnung der Absaugvorrichtung abdichtet. Die Stirnwand 63 liegt an einer Stirnseite 65 eines zylinderfömrigen Ansatzes 66 des Deckels 11 an.

Der zylinderförmige Ansatz 66 weist am Umfang zwei Öffnungen 70,71 auf. In der Verschlußstellung (Fig. 5) ist der drehbare Hohlzylinder 60 bezüglich des zylinderförmigen Ansatzes 66 so angeordnet, daß die Öffnungen 70,71 von der Zylinderwand des Hohlzylinders 60 verschlossen sind.

Zum Öffnen muß der Hohlzylinder 60 gegenüber des zylindrischen Ansatzes 66 um 90° verdreht werden, so daß Öffnungen 72,73 des Hohlzylinders 60 die Öffnungen 70,71 des zylindrischen Ansatzes 66 überdecken (Fig. 6). Hierzu wird der Zylinder 60 mit der Öffnungshilfe 62 verbunden, indem Ausnehmungen 74 des Hohlzylinders 60 in entsprechende Ansätze 75 der Öffnungshilfe 62 eingreifen. Da die Öffnungshilfe 62 fest mit dem Beckenboden 61 verbunden ist, ist der Hohlyzlinder 60 von der Öffnungshilfe 62 drehfest gehalten. Zum Öffnen wird nun der Behälter 10 aus der in Fig. 5 dargestellten Verschlußstellung um 90° in die in Fig. 6 dargestellte Öffnungsstellung verdreht. In der Öffnungsstellung fließt die Flüssigkeit aus dem Beutel 14 durch die Öffnungen 70,71 des zylindrischen Ansatzes 66, durch die Öffnungen 72,73 des Hohlzylinders 60 und durch eine Auslaßöffnung 76 des Hohlzylinders 60 in einen in dem Beckenboden 61 vorgesehenen Abfluß 77. Hierzu ist die Öffnungshilfe 62 über dem Abfluß 77 angeordnet. Um ein ungewolltes Verdrehen des Hohlzylinders 60 aus der Verschlußstellung zu vermeiden, können nicht dargestellte Rastnasen oder ähnliches vorgesehen sein.

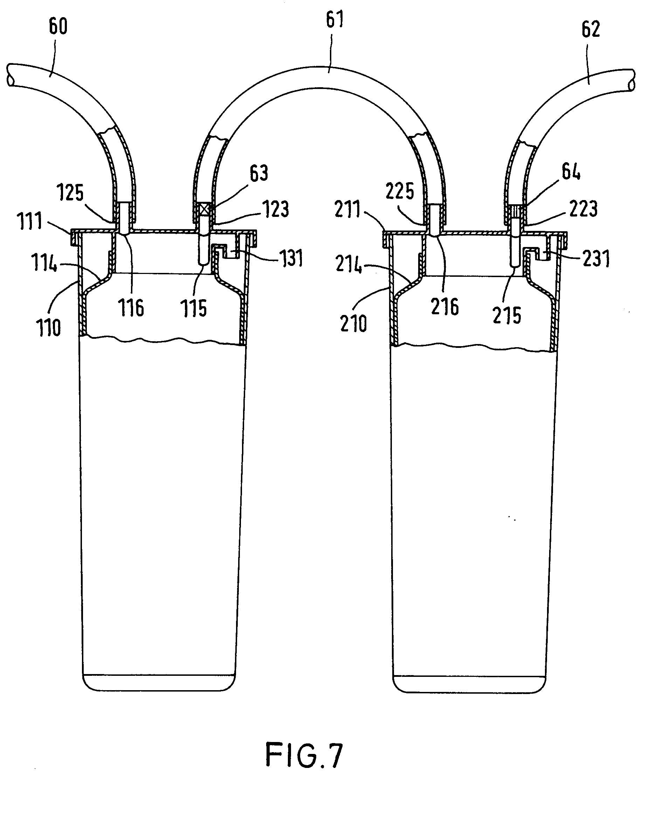

Fig. 7 zeigt eine Anordnung von zwei in Reihe geschalteten Absaugvorrichtungen, wie sie anhand der Fign. 1 bis 4 oder 5 und 6 beschrieben sind. Zur Verbesserung der Übersichtlichkeit ist der Ausguß nicht dargestellt.

Ein Anschlußstutzen 125 einer Einlaßöffnung 116 des ersten Behälters 110 ist mit einem Schlauch 60 verbunden. Der Schlauch 60 ist an einen Patienten angeschlossen. Ein Anschlußstutzen 123 einer Saugöffnung 115 ist mittels eines Schlauchs 61 mit einem Anschlußstutzen 225 einer Einlaßöffnung 216 eines zweiten Behälters 210 verbunden. Ein Anschlußstutzen 223 einer Saugöffnung 215 ist über einen Schlauch 62 mit einer Saugquelle verbunden.

Die Saugöffnungen 115,215 weisen jeweils im Deckel 111,211 eine Abzweigung 131,231 auf, die nicht mit dem Beutel 114 bzw. 214, sondern jeweils mit den Behältern 110,210 verbunden sind. Im Betrieb wird somit von der Saugquelle in dem Beutel 214 sowie über die Abzweigung 231 in dem Behälter 210 Unterdruck erzeugt. Aufgrund der Verbindung der beiden Behälter 210,110 über den Schlauch 61 wird in einem Beutel 114 und über eine Abzweigung 131 in dem Behälter 110 ebenfalls Unterdruck erzeugt. Nach Aufbau eines ausreichenden Unterdrucks wird über den Schlauch 60 Körperflüssigkeit angesaugt. Die angesaugte Körperflüssigkeit gelangt in den Beutel 114. Sobald der Beutel 114 gefüllt ist, wird die in dem Beutel 114 befindliche Körperflüssigkeit über den Schlauch 61 in den Beutel 214 transportiert. Der Anschlußstutzen 115 ragt in den Beutel 114 hinein, damit der Flüssigkeitsspiegel nicht über dieses Maß hinaus ansteigt und von diesem Niveau ausgehend der Beutel 214 über den Schlauch 61 befüllt wird. Um ein Zurückfließen der Körperflüssigkeit aus dem Schlauch 61 in den Beutel 114 zu vermeiden, ist in dem Anschlußstutzen 123 ein Rückschlagventil 63 vorgesehen. In dem Ansatzstutzen 222 des zweiten Behälters 210 ist entsprechend der anhand der Fign. 1 bis 4 beschriebenen Ansaugvorrichtung ein hydrophober Filter 64 vorgesehen. Mittels des hydrophoben Filters 64 ist sichergestellt, daß bei gefülltem Beutel 214 keine Flüssigkeit in den Schlauch 62 und somit zur Saugquelle gelangt.

Auf vorstehend beschriebene Weise kann auch eine größere Anzahl Absaugvorrichtungen in Reihe angeordnet werden. Hierbei ist mit Ausnahme des letzten mit der Saugquelle verbundenen Behälters in jedem Anschlußstutzen der Saugöffnungen ein Rückschlagventil und in dem letzten Anschlußstutzen des Behälters ein hydrophober Filter angeordnet.

## Patentansprüche

1. Absaugvorrichtung für Körperflüssigkeiten, mit einem luftdicht verschließbaren Behälter (10), in den ein flexibler Beutel (14) einsetzbar ist, mit einer Saugöffnung (15) zum Anschluß einer Saugquelle, einer an einem Patienten anschließbaren Einlaßöffnung (16) und einer Auslaßöffnung (17) zum Entleeren,
**dadurch gekennzeichnet,**
daß in der Auslaßöffnung (17) ein drehbarer oder axial bewegbarer Schieber (37,60) angeordnet ist, der bei auf dem Kopf stehendem Behälter durch Kontakt mit einem Beckenboden oder einer auf dem Beckenboden (61) befestigten Öffnungshilfe (62) betätigbar ist.

2. Absaugvorrichtung nach Anspruch 1, dadurch gekennzeichnet, daß der axial bewegbare Schieber (37) in eine Verschlußstellung aus dem Behälter (10) hervorragt und zum Öffnen in den Behälter (10) eindrückbar ist.

3. Absaugvorrichtung nach Anspruch 1 oder 2, dadurch gekennzeichnet, daß der Schieber (37,60) ein behälterseitig geschlossener Hohlkörper mit zumindest einer seitlich am Hohlkörper vorgesehenen Öffnung (40,41;72,73) und mit einer vom Behälter (10) abgewandten Ausgußöffnung (52,76) ist, so daß die Flüssigkeit bei betätigtem Hohlkörper durch die seitliche Öffnung (40,41;72,73), den Hohlkörper und die Ausgußöffnung (52,76) ausgießbar ist.

4. Absaugvorrichtung nach Anspruch 3, dadurch gekennzeichnet, daß die behälterseitige Fläche (38) des Hohlkörpers als Führungskörper (39) zum Leiten der Flüssigkeit ausgebildet ist.

5. Absaugvorrichtung nach einem der Ansprüche 1 bis 4, dadurch gekennzeichnet, daß der axial bewegbare Schieber (37) in eingedrücktem Zustand in gleicher Höhe endet wie an der Saug- (15) und Einlaßöffnung (16) vorgesehene Anschlußstutzen (23,25), so daß der Schieber (37) und die Anschlußstutzen (23,25) Stützen bilden, mit denen der Behälter (10) auf einer ebenen Fläche stehen kann.

6. Absaugvorrichtung nach einem der Ansprüche 1 oder 2-4, dadurch gekennzeichnet, daß die Höhe der Öffnungshilfe (62) auf den drehbaren Schieber (60) abgestimmt ist, so daß der Behälter (10) auf dem in die Öffnungshilfe (62) eingesetzten Schieber (60) und den Anschlußstutzen (23,25) sicher auf dem Kopf steht.

7. Absaugvorrichtung nach einem der Ansprüche 1 bis 6, dadurch gekennzeichnet, daß der Schieber (37) von einer Kappe (44) umschlossen ist, die mit dem Behälter (10) luftdicht abschließt.

8. Absaugvorrichtung nach Anspruch 7, dadurch gekennzeichnet, daß die Kappe (44) in die Ausgußöffnung (52) des Schiebers (37) hineinragende Rippen (47,48) aufweist, deren Außenabmessungen den Innenabmessungen des Schiebers (37) entsprechen.

9. Absaugvorrichtung für Körperflüssigkeiten, mit einem Behälter (10), der mittels eines Deckels (11) luftdicht verschließbar ist und in den ein mit dem Deckel (11) verbundener flexibler Beutel (14) einsetzbar ist, und der Deckel (11) eine Saugöffnung (15) zum Anschluß einer Saugquelle, eine an den Patienten anschließbaren Einlaßöffnung (16) und eine Auslaßöffnung (17) zum Entleeren aufweist,
**dadurch gekennzeichnet**,
daß in dem Deckel (11) eine Abzweigung (31) von der Saugleitung (15) vorgesehen ist, so daß über die Saugöffnung (15) gleichzeitig eine Verbindung zu dem Beutel (14) und über die Abzweigung (31) eine Verbindung zu dem Behälter (10) besteht.

10. Absaugvorrichtung nach Anspruch 9, dadurch gekennzeichnet, daß in der Saugöffnung (15) ein hydrophober Filter (30) oder ein Rückschlagventil angeordnet ist.

11. Anordnung mehrerer Absaugvorrichtungen nach Anspruch 9 oder 10 in Reihe, dadurch gekennzeichnet, daß jeweils die Einlaßöffnung (216) eines nachfolgenden Behälters (210) mit der Saugöffnung (115) eines vorhergehenden Behälters (110), die Einlaßöffnung (216) eines patientenseitigen ersten Behälters (110) mit dem Patienten und die Saugöffnung (215) des letzten Behälters (210) mit der Saugquelle verbunden ist, wobei in den mit Einlaßöffnungen (216) anderer Behälter verbundenen Saugöffnungen (115) ein Rückschlagventil (63) und in der Saugöffnung (215) des letzten Behälters (210) ein hydrophober Filter (64) vorgesehen ist.
